(19) Europäisches Patentamt
European Patent Office
Office européen des brevets

(11) Veröffentlichungsnummer: **0 302 833**
**A2**

(12) **EUROPÄISCHE PATENTANMELDUNG**

(21) Anmeldenummer: **88810530.1**

(22) Anmeldetag: **02.08.88**

(51) Int. Cl.[4]: **C 07 D 213/38**
C 07 D 213/61, A 01 N 43/40

(30) Priorität: **07.08.87 CH 3051/87 09.03.88 CH 888/88**

(43) Veröffentlichungstag der Anmeldung:
**08.02.89 Patentblatt 89/06**

(84) Benannte Vertragsstaaten:
**AT BE CH DE ES FR GB IT LI NL**

(71) Anmelder: **CIBA-GEIGY AG**
**Klybeckstrasse 141**
**CH-4002 Basel (CH)**

(72) Erfinder: **Gsell, Laurenz, Dr.**
**Maiengasse 56**
**CH-4056 Basel (CH)**

(54) **1-Nitro-2,2-diaminoäthylenderivate.**

(57) Neue 1-Nitro-2,2-diaminoäthylenderivate der Formel I

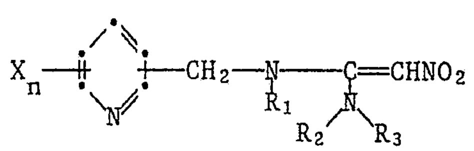

(I)

worin
X Chlor, Fluor, mit Halogen substituiertes oder unsubstituiertes $C_1$-$C_5$-Alkyl,
mit Halogen substituiertes oder unsubstituiertes $C_1$-$C_5$-Alkoxy,
mit Halogen substituiertes oder unsubstituiertes $C_1$-$C_5$-Alkylthio,
mit Halogen substituiertes oder unsubstituiertes Alkylsulphinyl,
mit Halogen substituiertes oder unsubstituiertes Alkylsulphonyl, ferner Nitro, Cyano, Thiocyanato, $C_3$-$C_5$-Haloalkenyl, $C_3$-$C_5$-Haloalkinyl, Hydroxy, $C_1$-$C_5$-Alkoxycarbonyl, Amino, Di-$C_1$-$C_4$-alkylamino, $C_1$-$C_5$-Alkylcarbonyl, $C_1$-$C_5$-Alkylcarbonylamino oder $C_1$-$C_5$-Alkylcarbonyloxy,
n die Zahlen 0-4,
$R_1$ Wasserstoff, $C_1$-$C_5$-Alkyl, $C_3$-$C_7$-Cycloalkyl,
$R_2$ Wasserstoff, $C_1$-$C_5$-Alkyl, $C_3$-$C_7$-Cycloalkyl,
$R_3$ Wasserstoff, $C_1$-$C_5$-Alkyl, $C_3$-$C_7$-Cycloalkyl, Benzyl oder Pyridinylmethyl, oder
$R_2$ und $R_3$ zusammen mit dem sie verbindenden Stickstoffatom der Pyrrolidinyl- oder den Piperazinylrest
bedeuten, wobei nicht mehr als einer der Reste $R_1$, $R_2$ oder $R_3$ für Wasserstoff steht, inklusive cis- und trans Isomere und Salze, Verfahren zur Herstellung dieser Verbindungen sowie diese enthaltende Mittel und deren Verwendung in der Schädlingsbekämpfung, insbesondere zur Bekämpfung von Insekten.

EP 0 302 833 A2

**Beschreibung**

### 1-Nitro-2,2-diaminoäthylenderivate

Die vorliegende Erfindung betrifft neue 1-Nitro-2,2-diaminoäthylenderivate, Verfahren und Zwischenprodukte zu ihrer Herstellung, Schädlingsbekämpfungsmittel, die diese Verbindungen enthalten, sowie ihre Verwendung in der Schädlingsbekämpfung.

Die erfindungsgemässen 1-Nitro-2,2-diamino-äthylenderivate entsprechen der Formel I

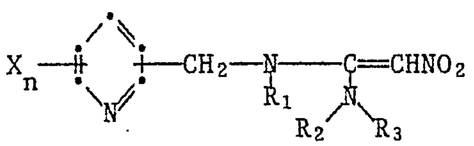 (I)

worin
X Chlor, Fluor, mit Halogen substituiertes oder unsubstituiertes $C_1$-$C_5$-Alkyl,
mit Halogen substituiertes oder unsubstituiertes $C_1$-$C_5$-Alkoxy,
mit Halogen substituiertes oder unsubstituiertes $C_1$-$C_5$-Alkylthio,
mit Halogen substituiertes oder unsubstituiertes Alkylsulphinyl,
mit Halogen substituiertes oder unsubstituiertes Alkylsulphonyl, ferner Nitro, Cyano, Thiocyanato, $C_3$-$C_5$-Haloalkenyl, $C_3$-$C_5$-Haloalkinyl,
Hydroxy, $C_1$-$C_5$-Alkoxycarbonyl, Amino, Di-$C_1$-$C_4$-alkylamino, $C_1$-$C_5$-Alkylcarbonyl, $C_1$-$C_5$-Alkylcarbonylamino oder $C_1$-$C_5$-Alkylcarbonyloxy,
n die Zahlen 0-4,
$R_1$ Wasserstoff, $C_1$-$C_5$-Alkyl, $C_3$-$C_7$-Cycloalkyl,
$R_2$ Wasserstoff, $C_1$-$C_5$-Alkyl, $C_3$-$C_7$-Cycloalkyl,
$R_3$ Wasserstoff, $C_1$-$C_5$-Alkyl, $C_3$-$C_7$-Cycloalkyl, Benzyl oder Pyridinylmethyl, oder
$R_2$ und $R_3$ zusammen mit dem sie verbindenden Stickstoffatom den Pyrrolidinyl- oder den Piperazinylrest bedeuten, wobei nicht mehr als einer der Reste $R_1$, $R_2$ und $R_3$ für Wasserstoff steht, einschliesslich der cis- und trans-Isomere und einschliesslich der Salze der Verbindungen der Formel I.

Die als Substituenten in Betracht kommenden Alkyle und alkylhaltigen Reste, wie Alkoxy, Alkylthio, Alkylsulfinyl oder Alkylsulfonyl können geradkettig oder verzweigt sein. Als Beispiele solcher Alkyle seien Methyl, Aethyl, Propyl, Cyclopropyl, Isopropyl, Butyl, i-Butyl, sek.Butyl, tert.Butyl oder Pentyl, und ihre Isomeren genannt.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden durch Halogen substituierten $C_1$-$C_5$-Alkyle können geradkettig oder verzweigt sein und nur teilweise oder auch perhalogeniert sein, wobei für die Halogene und die Alkyle die oben gegebenen Definitionen gelten. Geeignete Beispiele solcher Substituenten sind u.a. das ein- bis dreifach durch Fluor, Chlor und/oder Brom substituierte Methyl wie z.B. $CHF_2$ oder $CF_3$; das ein- bis fünffach durch Fluor, Chlor und/oder Brom substituierte Aethyl wie z.B. $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie z.B. $CH_2CHBrCH_2Br$, $CF_2CHFCF_3$, $CH_2CF_2CF_3$ oder $CH(CF_3)_2$; das ein- bis neunfach durch Fluor, Chlor und/oder Brom substituierte Butyl oder eines seiner Isomeren wie z.B. $CF(CF_3)CHFCF_3$ oder $CH_2(CF_2)_2CF_3$.

Als Pyridinylmethylrest kommt in den Verbindungen der Formel I jedes der drei Isomere α, β und γ der Formel:

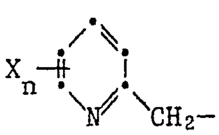
α

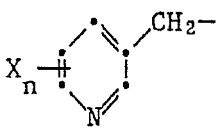
β

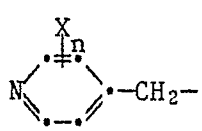
γ

in Betracht.

Die Verbindungen der Formel I können je nach sterischer Anordnung der an den Aethylenkohlenstoffatomen gebundenen Substituenten als eines der cis-und trans-Isomeren oder als deren Gemisch vorliegen.

Wenn einer der Reste $R_1$, $R_2$ und $R_3$ Wasserstoff bedeutet, kann die erfindungsgemässen Enamine auch in der tautomeren Imin-Form vorliegen.

Die Verbindungen der Formel I können auch in Form von Säureadditionssalzen vorliegen. Zur Bildung solcher Salze eignen sich sowohl organische als auch anorganische Säuren. Beispiele solcher Säuren sind u.a. Chlorwasserstoffsäure, Bromwasserstoffsäure, Salpetersäure, verschiedene Phosphorsäuren, Schwefelsäure, Essigsäure, Propionsäure, Buttersäure, Valeriansäure, Oxalsäure, Malonsäure, Maleinsäure, Fumarsäure, Milchsäure, Weinsäure oder Salicylsäure.

Im Rahmen der vorliegenden Erfindung sind folgende Verbindungsgruppen der Formel I bevorzugt.

1. Jene 1-Nitro-2,2-diamino-äthylenderivate, bei welchen
X Chlor oder Fluor bedeuten.

2. Jene 1-Nitro-2,2-diamino-äthylenderivate, bei welchen
X Chlor,
n die Zahlen 0-2,
$R_1$ Wasserstoff, Methyl, Aethyl oder Cyclopropyl,
$R_2$ Wasserstoff oder Methyl,
$R_3$ Wasserstoff, $C_1$-$C_5$-Alkyl, Benzyl oder Pyridinylmethyl, oder
$R_2$ und $R_3$ zusammen mit dem sie verbindenden Stickstoffatom den Pyrrolidinyl- oder den Piperazinylrest, bedeuten.

2.1 Von diesen Verbindungen sind jene der Formel Ia

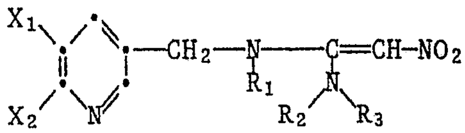

(Ia)

bevorzugt bei welchen $X_1$ und $X_2$ voneinander unabhängig Wasserstoff oder Chlor und $R_1$ Wasserstoff, Methyl oder Aethyl bedeuten.

2.1.1. Von dieser Gruppe sind jene hervorzuheben, in welchen n = 0 ist.
Von der letztgenannten Gruppe sind folgende Einzelverbindungen hervorzuheben:

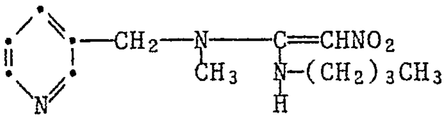
,

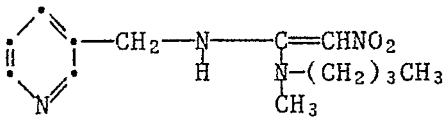
,

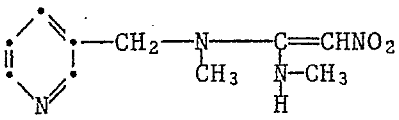
,

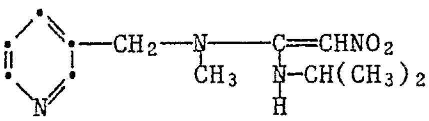
,

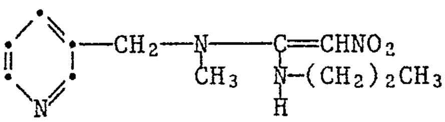
,

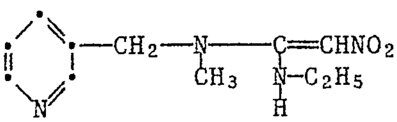
,

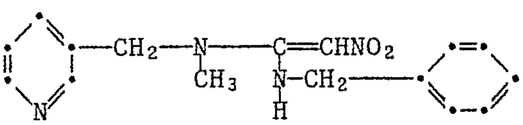
,

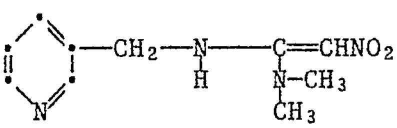
,

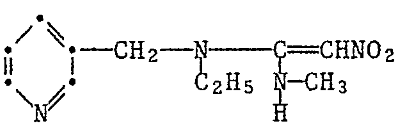
,

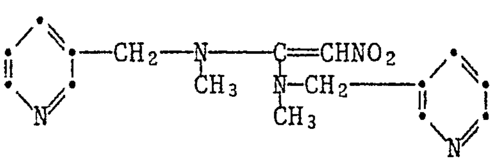
,

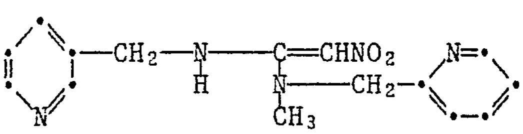
.

2.1.2 Von den unter Ziffer 2.1 definierten Verbindungen der Formel Ia sind auch noch jene bevorzugt, in welchen $X_1$ und $X_2$ Chlor bedeuten.
Von dieser Gruppe ist insbesondere die Verbindung der Formel

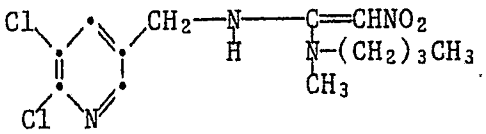

bevorzugt.

Schliesslich sind von den unter Ziffer 2 definierten Verbindungen jene der Formel Ic

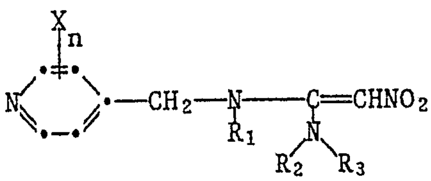

(Ic)

bevorzugt.

Die erfindungsgemässen Verbindungen der Formel I können durch stufenweise Umsetzung der Nitromethylenderivate der Formel II

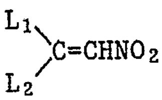

(II),

in welcher $L_1$ und $L_2$ Abgangsgruppen, wie beispielsweise Alkylthio, Alkylsulfinyl oder Chlor bedeuten, mit zweckmässig substituierten Aminen hergestellt werden.

Dabei wird die Verbindung der Formel II, worin $L_1$ und $L_2$ die obengenannte Bedeutung haben, entweder

a) zunächst mit der äquivalenten Menge von einem Pyridinylmethylamin der Formel III

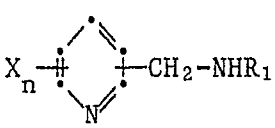

(III),

worin $R_1$ die unter Formel I angegebene Bedeutung hat, zum 1-Nitro-2-pyridinylmethylamino-äthylenderivat der Formel IV

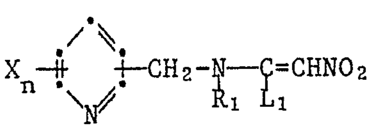

(IV),

worin $R_1$, X und n, bzw. $L_1$ die unter den Formel I bzw. II angegebene Bedeutung haben, umgesetzt und anschliessend die Verbindung IV mit einem Amin der Formel V

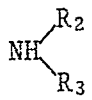

(V),

worin $R_2$ und $R_3$ die unter Formel I angegebene Bedeutung haben, zum 1-Nitro-2,2-diamino-äthylenderivat der Formel I umgesetzt, oder

b) ein Nitroäthylenderivat der Formel II wird zunächst mit der äquivalenten Menge von einem Amin der Formel V zum 1-Nitro-2-amino-äthylenderivat der Formel VI,

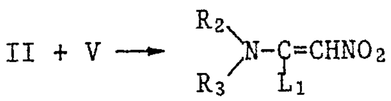

(VI),

worin $R_2$ und $R_3$, bzw. $L_1$ die unter Formel II bzw. V angegebene Bedeutung haben, umgesetzt und anschliessend wird diese Verbindung mit einem Pyridinylmethylamin der Formel III zum 1-Nitro-2,2-diamino-äthylenderivat der Formel I umgesetzt und dieses isoliert.

Nach einer bevorzugten Ausführungsform des Herstellungsverfahrens wird das jeweilige Zwischenprodukt der Formel IV oder der Formel VI ohne Isolierung weiterverarbeitet.

Die Zwischenprodukte der Formel IV sind neu, zeigen starke insektizide Eigenschaften und sind ebenfalls Gegenstand der vorliegenden Erfindung. Die Verbindungen der Formel IV können in der ersten Stufe des oben beschriebenen Verfahrens hergestellt und anschliessend in Substanz isoliert werden.

Das Verfahren zur Herstellung der Verbindungen der Formel I über die neuen Zwischenprodukte der Formel IV ist ebenfalls Gegenstand der vorliegenden Erfindung, genauso wie das über die bekannten Zwischenprodukte der Formel VI zu den Verbindungen der Formel I führende Verfahren, sowie das Verfahren zur Herstellung der Verbindung der Formel IV.

Als Lösungsmittel bei der Herstellung der Zwischenprodukte IV und VI, sowie des Endprodukts I eignen sich aprotische, polare Lösungsmittel, wie Acetonitril, Dimethylformamid, Dimethylsulfoxid, usw., sowie aromatische Lösungsmittel, wie Benzol oder Toluol, ferner Chlorkohlenwasserstoffe, Tetrahydrofuran, oder Aether und Alkohole.

Die Umsetzungen verlaufen im Temperaturbereich zwischen 0° und dem Kochpunkt des Reaktionsgemisches, unter atmospherischem oder gegebenenfalls unter vermindertem Druck.

Der Zusatz von Salzen, welche als Puffer wirken, wie zum Beispiel Dinatriumhydrophosphat können den Reaktionsverlauf begünstigen.

Die Ausgangsprodukte der Formel II sind bekannt und können, genauso wie die Zwischenprodukte der Formel VI, nach bekannten Methoden hergestellt werden.

Von den Verbindungen der Formel II, in welchen $L_1$ und $L_2$ Niederalkylthio oder Benzylthio bedeuten, sind bekannte Zwischenprodukte der präparativen organischen Chemie und sind im Handel käuflich.

Jene Verbindungen der Formel II, in welchen $L_1$ und $L_2$ Chlor bedeuten, können nach EP-A 135 803 aus dem bekannten 1-Nitro-2-trichloräthan durch HCl-Abspaltung hergestellt werden.

Jene Verbindungen der Formel II in welchen $L_1$ einen Alkylthiorest und $L_2$ einen Alkylsulfinylrest bedeuten, sind in der US-Patentschrift US 4,028,375 beschrieben.

Die Zwischenprodukte der Formel VI, sowie deren Herstellung sind in der Gazetta Chimica Italiana 111 217 (1981) und im Arch. Pharm. (2) 161-167 (1986) beschrieben.

Die Verbindungen der Formel

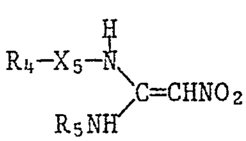

in welcher

$R_4$ für einen Aryl- oder Heteroarylrest steht, der gegebenenfalls substituiert ist,

$R_5$ für geradkettiges, verzweigtes oder cyclisches Alkyl oder Alkenyl, das gegebenenfalls durch Alkoxy oder Cycloalkyl substituiert ist, steht oder für einen gegebenenfalls substituierten Aryl- oder Heteroarylrest steht,

$X_5$ für gegebenenfalls alkylsubstituiertes Methylen oder eine chemische Bindung steht, wobei $R_5$ nicht gleich Aryl ist, wenn $X_5$ eine Einfachbindung darstellt, sind als kreislufbeeinflussende Arzneimittel, insbesondere als blutdrucksenkende Mittel aus der DE-OS 32 32 462 bekannt.

In der EP-A 154 178 werden insektizid, mitizid und nematizid wirksame 1-Pyridinylalkyl-2-nitromethyliden-1,3-diazacycloalkane beschrieben, wobei der Heterocyclus 5-7 Glieder aufweist. Diese Veröffentlichung betrifft Verbindungen, welche der Formel

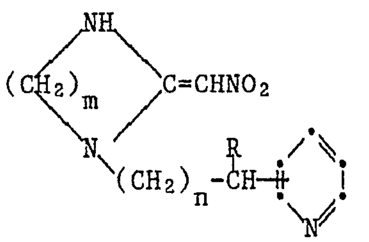

entsprechen, wobei für m 2, 3 oder 4 und n 0, 1, 2 oder 3 steht.

Aehnliche, ebenfalls insektizid wirksame 1-Pyridinylmethyl-2-nitromethyliden-azacycloalkane und 1-Pyridinylmethyl-2-nitromethyliden-1,3-diazacycloalkane wurden auch in der EP-A 192 060 beschrieben.

Als Unterschied zu den in diesen Publikationen beschriebenen Verbindungen, bei welchen das 2-ständige Aethylen-Kohlenstoffatom als Glied eines Heterocyclus definiert ist, ist der entsprechende Rest der erfindungsgemässen Verbindungen nicht ringförmig.

Andere, ebenfalls insektizid wirksame Nitroäthylenderivate sind aus den "Advances in Pesticide Science", Part 2, Pergamon Press, 1979, S. 206-217 bekannt. Diese Verbindungen enthalten jedoch keinen Pyridinylmethylrest.

Ziel der vorliegenden Erfindung war, weitere Aktivsubstanzen für die Schädlingsbekämpfung bereitzustellen.

Ueberraschenderweise wurde gefunden, dass die erfindungsgemässen Verbindungen der Formel I, sowie die Zwischenprodukte der Formel IV bei günstiger Warmblüter- und Pflanzenverträglichkeit wertvolle Wirkstoffe in der Schädlingsbekämpfung sind. So eignen sich die Verbindungen der Formeln I und IV z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formeln I und IV von Imagines aufgenommen, so kann sich ihre Wirkung in unmittelbarer Abtötung der Schädlinge zeigen oder aber in verminderter Eiablage und/oder Schlupfrate. Die letztere Erscheinung kann inbesondere bei Coleopteren beobachtet werden. Bei der Bekämpfung von tierparasitären Schädlingen, insbesondere an Haus-und Nutztieren, kommen vor allem Ektoparasiten, wie z.B. Milben und Zecken und Dipteren, wie z.B. Lucilia sericata in Betracht.

Die gute pestizide Wirkung der erfindungsgemässen Verbindungen der Formeln I und IV entspricht einer Abtötungsrate (Mortalität) von mindestens 50-60 % der erwähnten Schädlinge.

Die Verbindungen der Formeln I und IV werden in unveränderter Form oder vorzugsweise zusammen mit in der Formulierungstechnik üblichen, inerten und pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formeln I und/oder IV, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der

physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder IV oder der Kombinationen dieser Wirkstoffe mit andern Insektiziden oder Akariziden nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze oder Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einem Alkylrest mit 8 bis 22 C-Atomen enthalten und als weitere Substituenten niedrige, gegebenenfalls halogenierte Alkyl-, Benzyl- oder niedrige Hydroxyalkyl reste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder IV oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## 1. Herstellung der Zwischen- und Endprodukte

### 1.1 Zwischenprodukte der Formel IV

Beispiel 1.1.1: Herstellung des Zwischenproduktes
2-(N-Methyl-N-pyridin-3-yl-methyl-amino)-2-methylthio-1-nitro-äthylen

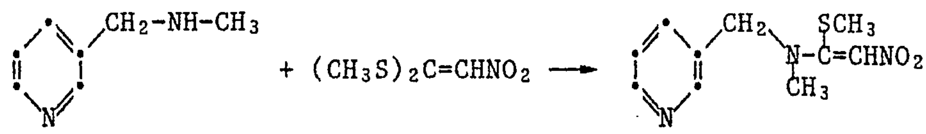

26 g (0,156 Mol) 1-Nitro-2,2-dimethylthioäthylen werden in 80 ml Toluol gelöst. Zu dieser Lösung werden 18,3 g (0,15 Mol) N-Methyl-N-pyridin-3-yl-methylamin bei 70°C während 30 Min. zugetropft und die Lösung wird bei derselben Temperatur eine Stunde lang weitergerührt.

Nach Verdampfen des Lösungsmittels wird der Verdampfungsrückstand in Lösung (Aethylenacetat/Methanol = 97/3) über Kieselgel säulenchromatographisch gereinigt. Das 2-(N-Methyl-N-pyridin-3-yl-methyl-amino)-2-methylthio-1-nitro-äthylen (Produkt Nr. 1.1, Tabelle 1) wird nach Verdampfen des Lösungsmittels in Vakuum als dickes Oel isoliert.

In analoger Weise können die folgenden Verbindungen hergestellt werden:

Tabelle 1

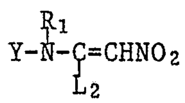

| Verb. Nr. | Y | $L_2$ | $R_1$ | Phys. konst. |
|---|---|---|---|---|
| 1.1 | 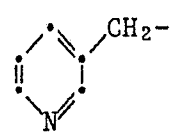 | $CH_3S-$ | $CH_3-$ | dickes Oel |
| 1.2 | 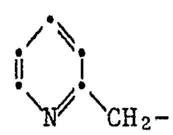 | $CH_3S-$ | H- | Smp. 136-138° |
| 1.3 | 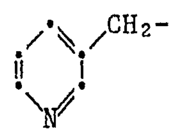 | $CH_3S-$ | H- | Smp. 124-126° |

Beispiel 1.2.1: Herstellung des Zwischenprodukts 2-(N-Butyl-N-methyl-amino)-2-methylthio-1-nitro-äthylen.

Die toluolische Lösung von 8,26 g (0,05 Mol) 1-Nitro-2,2-di(methyl-thio)-äthylen und 5,25 g (0,06 Mol) N-n-Butyl-N-methylamin werden während 2 Stunden unter Rückfluss erhitzt. Nach Verdampfen des Toluols in Vakuum werden als Destillationsrückstand 10,2 g 2-(N-Butyl-N-methyl-amino)-2-methylthio-1-nitro-äthylen als Oel isoliert ($n_D^{21}$ = 1,584). (Produkt Nr. 2.1, Tabelle 2.)

In analoger Weise können die folgenden Verbindungen hergestellt werden:

Tabelle 2

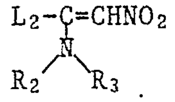

| Verb. Nr. | $L_2$ | $R_1$ | $R_3$ | Phys. konst. |
|---|---|---|---|---|
| 2.1 | $CH_3S-$ | $CH_3-$ | $n-C_4H_9-$ | $n_D^{21} = 1,584$ |

1.3 Endprodukte

Beispiel 1.3.1: Herstellung des Endproduktes 2-Butylamino-2-(N-methyl-N-pyridin-3-yl-methyl-amino)-1-nitro-äthylen.

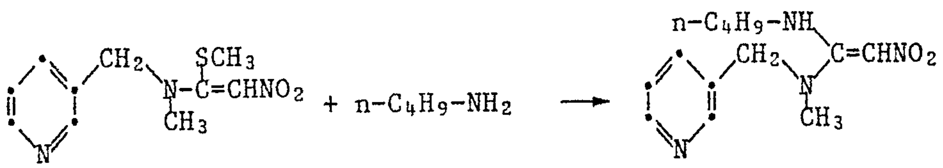


6 g 2-(N-Methyl-N-pyridin-3-yl-methyl-amino)-2-methylthio-1-nitro-äthylen, 2,19 g n-Butylamin in 20 ml Acetonitril als Lösungsmittel werden während 4 Stunden bei 70°C gehalten. Nach Verdampfen des Lösungsmittels in Vakuum wird der Verdampfungsrückstand in Dichlormethan gelöst und auf Kieselgel säulenchromatographiert. Das Produkt wird mit Dichlormethan/Methanol = 95/5 eluiert. Nach Verdampfen des Lösungsmittels wird das 2-Butylamino-2-(N-methyl-N-pyridin-3-yl-methyl-amino)-1-nitro-äthylen als Oel isoliert, ($n_D^{22} = 1,618$). (Produkt Nr. 3.1, Tabelle 3.)

Beispiel 1.3.2: Herstellung des 2-(N-n-Butyl-N-methyl-amino)-2-(pyridin-3-yl-methyl-amino)-1-nitro-äthylen.

5,2 g 2-(N-n-Butyl-N-methyl-amino)-2-methylthio-1-nitro-äthylen, 2,84 g Pyridin-3-ylmethylamin, 3,69 g Dinatriumhydrophosphat in 30 ml Aethanol werden 1 Stunde lang unter Rückfluss erhitzt. Nach Filtrieren des Salzes wird das Lösungsmittel aus der Lösung in Vakuum verdampft und der Verdampfungsrückstand in Aethylacetat/ Methanol = 95/5 über eine mit Kieselgel gefüllte Säule chromatographiert. Das Produkt wird mit Aethylacetat/Methanol 85/15 eluiert; nach Verdampfen des Lösungsmittels werden 3,7 g 2-(N-n-Butyl-N-methyl-amino)-2-(pyridin-3-yl-methyl-amino)-1-nitro-äthylen als dickflüssiges Oel isoliert (Produkt 3.2, Tabelle 3).

Das NMR-Spektrum und die Elementaranalyse sind in Uebereinstimmung mit der genannten Struktur.

Beispiel 1.3.3: Herstellung des Endproduktes 2-Methylamino-2-(N-methyl-N-pyridin-3-yl-methyl-amino)-1-nitro-äthylen.

5,0 g 2-(N-Methyl-N-pyridin-3-yl-methyl-amino)-2-methylthio-1-nitro-äthylen und 2,07 g 33 %-ige Methylaminlösung in Aethanol werden mit 3,12 g Dinatriumhydrophosphat und 25 ml Aethanol während 1 Std. am Rückfluss erhitzt. Nach Abfiltrieren der Salze wird das Filtrat in Vak. verdampft und der Verdampfungsrückstand in Aethylacetat/ Methanol = 95/5 bis 85/5 über Kieselgel säulenchromatographiert. Das Produkt wird nach Verdampfen der Lösungsmittel als dickflüssiges Oel isoliert (Produkt 3.3, Tabelle 3).

In analoger Weise können die folgenden Verbindungen hergestellt werden:

Tabelle 3

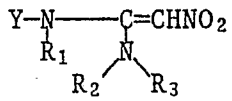

| Nr. | Y | $R_1$ | $R_2$ | $R_3$ | Phys. Konst. |
|---|---|---|---|---|---|
| 3.1 | Z* | $CH_3$- | H- | n-$C_4H_9$- | $n_D^{22}$: 1,618 |
| 3.2 | Z* | H- | $CH_3$ | n-$C_4H_9$- | dickes Oel |
| 3.3 | Z* | $CH_3$- | H- | $CH_3$ | dickes Oel |
| 3.4 | Z* | $CH_3$- | H- | $(CH_3)_2CH$- | Smp. 103-113° |
| 3.5 | Z* | $CH_3$- | H- | n-$C_3H_7$- | dickes Oel |
| 3.6 | Z* | $CH_3$- | H- | $C_2H_5$- | Oel |
| 3.7 | Z* | $CH_3$- | H- | $C_6H_5CH_2$- | Smp. 100,5-102° |
| 3.8 | Z* | $CH_3$- | $CH_3$- | Z* | dickes Oel |
| 3.9 | Z* | H- | $-(CH_2)_4-$ | | fest |
| 3.10 | Z* | H- | $-(CH_2)_5-$ | | dickes Oel |
| 3.11 | Z* | H- | $CH_3$- | $CH_3$- | dickes Oel |
| 3.12 | Z* | H- | $CH_3$- | n-$C_4H_9$ | dickes Oel |
| 3.13 | 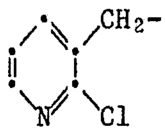 | H- | $CH_3$- | n-$C_4H_9$- | |
| 3.14 | 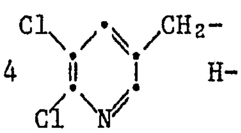 | H- | $CH_3$- | n-$C_4H_9$- | |
| 3.15 | Z* | H- | $CH_3$- | 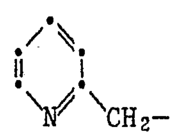 | |
| 3.16 | Z* | $CH_3CH_2$- | H- | n-$C_4H_9$- | Harz |
| 3.17 | Z* | 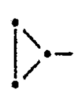 | H- | $CH_3$- | 114-116° |
| 3.18 | Z* | 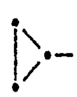 | H- | n-$C_4H_9$- | $n_D^{24}$: 1.6040 |
| 3.19 | Z* | 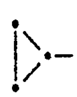 | H- | n-$C_6H_5CH_2$- | $n_D^{24}$: 1.6258 |
| 3.20 | Z* | $CH_3CH_2$- | H- | Z* | Harz |
| 3.21 | Z* | n-$C_4H_9$- | H- | $CH_3$- | Harz |

Tabelle 3 (Fortsetzung)

| Nr. | Y | $R_1$ | $R_2$ | $R_3$ | Phys. Konst. |
|---|---|---|---|---|---|
| 3.22 | Z* | $CH_3CH_2-$ | H- | $CH_3-$ | Harz |
| 3.23 | Z*** | $n-C_3H_7-$ | H- | $CH_3-$ | Harz |
| 3.24 | Z*** | $CH_3-$ | H- | $CH_3-$ | Harz |
| 3.25 | Z*** | $n-C_4H_9$ | H- | $n-C_3H_7-$ | Harz |
| 3.26 | Z** | $CH_3-$ | H- | $n-C_4H_7-$ | Harz |

Z* = 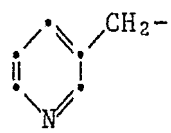

Z** = 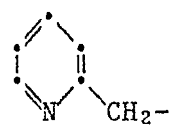

Z*** = 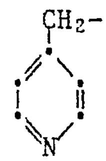

Beispiel 2: Formulierungen von Wirkstoffen der Formeln I und IV gemäss den Herstellungsbeispielen 1.1.1. und 1.3.1 bis 1.3.3

(% = Gewichtsprozent)

| 2.1 Emulsions-Konzentrate | a) | b) |
|---|---|---|
| Wirkstoff gemäss den Herstellungsbeispielen 1.1.1. und 1.3.1-1.3.3. | 10 % | 25 % |
| Ca-Dodecylbenzolsulfonat | - | 5 % |
| Ricinusöl-polyäthylenglykoläther (36 Mol AeO) | 25 % | 5 % |
| Tributylphenol-polyäthylenglykoläther (30 Mol AeO) | - | - |
| Cyclohexanon | - | 40 % |
| Butanol | 15 % | - |
| Xylolgemisch | - | 25 % |
| Essigester | 50 % | - |

Aus solchen Konzentraten können durch Verdünnen mit Wasser Emulsionen jeder gewünschten Konzentration hergestellt werden.

| 2.2 Lösungen | a) | b) |
|---|---|---|
| Wirkstoff gemäss den Herstellungsbeispielen 1.1.1. und 1.3.1. bis 1.3.3. | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190°C) | - | 74 % |

Die Lösungen sind zur Anwendung in Form kleinster Tropfen geeignet.

| 2.3 Granulate | a) | b) |
|---|---|---|
| Wirkstoff gemäss den Herstellungsbeispielen 1.1.1. und 1.3.1. bis 1.3.3. | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4 Extruder Granulat

Wirkstoff gemäss den Herstellungsbeispielen 1.1.1. und 1.3.1-1.3.3. 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Diese Gemisch wird extrudiert und anschliessend im Luftstrom getrocknet.

2.5 Umhüllungs-Granulat

Wirkstoff gemäss den Herstellungsbeispielen 1.1.1. und 1.3.1-1.3.3. 3 %
Polyäthylenglykol (MG 200) 3 %
Kaolin 94 %

Der feingemahlene Wirkstoff wird in einem Mischer auf das mit Polyäthylenglykol angefeuchtete Kaolin gleichmässig aufgetragen. Auf diese Weise erhält man straubfreie Umhüllungs-Granulate.

| 2.6 Stäubemittel | a) | b) | c) | d) |
|---|---|---|---|---|
| Wirkstoff gemäss den Herstellungsbeispielen 1.1.1. und 1.3.1.-1.3.3. | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

| 2.7 Spritzpulver | a) | b) | c) |
|---|---|---|---|
| Wirkstoff gemäss den Herstellungsbeispielen 1.1.1. und 1.3.1.-1.3.3. | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

2.8 Suspensions-Konzentrat

Wirkstoff gemäss den Herstellungsbeispielen 1.1.1. und 1.3.1.-1.3.3. 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3.1:

Frassgift- und Kontakt-Wirkung auf Laodelphax striatellus und Nilaparvata lugens (Nymphen);

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit 100 ml einer, aus dem Emulsionskonzentrat gemäss Beispiel 2a) durch Wasserzusatz hergestellten Emulsion, enthaltend 400 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortilität erfolgt 1, 4 und 8 Tage nach der Behandlung.

Die Verbindungen Nr. 1.1, 3.1, 3.2, 3.3, 3.4, 3.5, 3.6, 3.7 und 3.8 zeigen 80-100 % Wirkung in diesem Test gegen Nilaparvate lugens.

Beispiel 3.2:

Systemische Wirkung auf Nilaparvata lugens

Etwa 10 Tage alte Reispflanzen (ca. 10 cm hoch) werden in einen Plastik-Becher eingestellt, der 20 ml einer, aus dem Emulsionskonzentrat gemäss Beispiel 2a) durch Wasserzusatz hergestellten, wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wurde dann mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im N 2 bis N 3 Stadium besiedelt und mit einem Plastikzylinder abgedeckt. Der Versuch wird bei 20°C und 60 % relativer Luftfeuchtigkeit mit einer Beleuchtungsperiode von 16 Stunden durchgeführt. Nach fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Die Verbindungen Nr. 1.1, 3.1, 3.2, 3.3, 3.4, 3.5, 3.6, 3.7 und 3.8 zeigen im obigem Test 80 - 100%ige Wirkung (Mortalität) gegen Nilaparvata lugens.

3.3 Wirkung auf Spodoptera littoralis (Larven)

Es werden zwei in Töpfen gezogene Baumwollpflanzen von ca. 15-20 cm Höhe mit einer sprühfähigen Zubereitung des zu prüfenden Wirkstoffes behandelt. Nach Antrocknen des Sprühbelages werden die eingetropften Pflanzen in ein Blechgefäss von etwa 20 Litern Inhalt gestellt, das mit einer Glasplatte abgedeckt wird. Die Feuchtigkeit im Inneren des abgedeckten Gefässes wird so reguliert, dass sich kein Kondenswasser bildet. Direktes, auf die Pflanzen fallendes Licht wird vermieden. Dann werden die zwei Pflanzen infestiert, und zwar insgesamt mit:

a) 50 Larven von Spodoptera littoralis oder Heliothis virescens des ersten larvalen Stadiums;

b) 20 Larven von Spodoptera littoralis oder Heliothis virescens des dritten larvalen Stadiums; Nach 2 Tagen erfolgt die Auswertung gegenüber unbehandelten Kontrollen auf Anzahl der noch lebenden Larven.

Die Verbindung Nr. 3.3 zeigt in einer Konzentration von 400 ppm eine Mortalität von 80-100 %.

3.4 Wirkung auf Heliothis virescens (Eier)

Eine im Topf gezogene Baumwollpflanze wird von ca. 15-20 cm Höhe mit einer sprühfähigen flüssigen Zubereitung des zu prüfenden Wirkstoffes behandelt. Nach Antrocknen des Sprühbelages wird die eingetopfte Pflanze in ein Blechgefäss von etwa 20 Litern Inhalt gestellt, das mit einer Glasplatte abgedeckt wird. Die Feuchtigkeit im Inneren des abgedeckten Gefässes wird so reguliert, dass sich kein Kondenswasser bildet. Direktes, auf die Pflanze fallendes Licht wird vermieden. Dann wird die Pflanze infestiert, und zwar insgesamt mit zwei Eispiegeln von Spodoptera littoralis oder Heliothis virescens (dazu werden je 2 Blätter einer Pflanze in einem beidseitig mit Gaze verschlossenen Plexiglaszylinder eingeschlossen); zwei Eispiegel von Spodoptera oder ein Teil eines Baumwollblattes mit darauf abgelegten Eiern von Heliothis werden zu den eingeschlossenen Blättern gegeben.

Nach 4 Tagen erfolgt die Auswertung gegenüber unbehandelter Kontrolle unter Berücksichtigung der Schlupfrate (Anzahl der aus den Eiern geschlüpften Larven).

Die Verbindungen Nr. 3.3 und 3.6 zeigen in einer Konzentration von 400 ppm 100 % Mortalität in obigem Test.

3.5. Kontaktwirkung auf Myzus persicae

Etwa 4 bis 5 Tage alte, in Wasser angezogene Erbsenkeimlinge (pisum sativum) werden vor Versuchsbeginn jeweils mit ca. 200 Individuen der Spezies Myzus persicae besiedelt. Die so behandelten Pflanzen werden 24 Stunden später mit einer wässrigen Suspension enthaltend bis zu 200 ppm der zu prüfenden Verbindung bis zur Tropfnässe direkt besprüht. Man verwendet pro Testsubstanz zwei Pflanzen. Eine Auswertung der erzielten Abtötungsrate erfolgt 24 und 72 Stunden nach Applikation. Der Versuch wird bei 21-22°C und etwa 60 % relativer Luftfeuchtigkeit durchgeführt.

Die Verbindungen Nr. 1.1, 3.1, 3.2, 3.3, 3.4, 3.5, 3.6, 3.7 und 3.8 zeigen gute Wirkung in diesem Test.

3.6 Kontaktwirkung auf Aphis craccivora

In Töpfen angezogene 4-5 Tage alte Erbsenkeimlinge (pisum sativum) werden vor Versuchsbeginn mit je ca. 200 Individuen der Spezies Aphis craccivora besiedelt. Die so behandelten Pflanzen werden 24 Stunden später mit einer wässrigen Zubereitung enthaltend die zu prüfenden Verbindung bis zur Tropfnässe direkt besprüht. Man verwendet pro Test-Verbindung zwei Pflanzen. Eine Auswertung der erzielten Abtötungsrate erfolgt nach weiteren 24 und 72 Stunden. Der Versuch wird bei 21-22°C und einer rel. Luftfeuchtigkeit von etwa 55 % durchgeführt.

Die Verbindungen Nr. 1.1, 3.1, 3.2, 3.3, 3.4, 3.5, 3.6, 3.7 und 3.8 zeigen gute Wirkung in diesem Test.

3.7. Wirkung gegen Bodeninsekten (Diabrotica balteata)

Es werden 5 etwa einen bis drei cm lange Maiskeimlinge sowie eine Rondelle aus Filterpapier in eine 0,2 bis 12,5 ppm des zu prüfenden Wirkstoffes enthaltende wässrige Zubereitung getaucht. Die feuchte Filterpapierrondelle wird auf dem Boden eines 200 ml Plastik-bechers ausgelegt, und dann werden die 5 behandelten Maiskeimlinge zusammen mit 10 Larven von Diabrotica balteata des zweiten bis dritten Larvalstadiums in den Becher gegeben. Pro Wirkstoffkonzentration werden 2 Ansätze durchgeführt. Die mit den Larven besetzten Becher werden während 6 Tagen bei Tageslicht, einer relativen Luftfeuchtigkeit von 40 bis 60 % und einer Temperatur von 22 bis 24° C gehalten. Danach wird die prozentuale Abtötung der Testtiere bestimmt.

Die Verbindungen Nr. 1.1, 3.1, 3.2, 3.3, 3.4, 3.5, 3.6, 3.7 und 3.8 zeigen in diesem Test gute Wirkung.

3.8 Insektizide systemische Wirkung: Aphis craccivora

Bewurzelte Bohnenpflanzen werden in Töpfe, welche 600 ccm Erde enthalten, verpflanzt. Anschliessend giesst man 50 ml einer Zubereitung der zu prüfenden Verbindungen (erhalten aus einem 25%igen Spritzpulver) jeweils in einer Konzentration von 400 ppm direkt auf de Erde in den Töpfen.

Nach 24 Stunden werden auf die oberirdischen Pflanzenteile Läuse der Spezies Aphis craccivora gesetzt und die Pflanzen mit einem Plastikzylinder überstülpt, um die Läuse vor einer eventuellen Kontakt-oder Gaswirkung der Testsubstanz zu schützen.

Die Auswertung der erzielten Abtötung erfolgt 48 und 72 Stunden nach Versuchsbeginn. Pro Testsubstanz werden zwei Pflanzen, je eine in einem separaten Topf, verwendet. Der Versuch wird bei 25° C und 70 % relativer Luftfeuchtigkeit durchgeführt.

Die Verbindungen Nr. 1.1, 3.1, 3.2, 3.3, 3.4, 3.5, 3.6, 3.7, und 3.8 zeigen gute Wirkung in obigem Test.

3.9 Insektizide systemische Wirkung: Myzus persicae

Bewurzelte Kohlpflanzen im 4- bis 5-Blatt-Stadium werden in Töpfe, welche 60 ccm Erde enthalten, verpflanzt. Anschliessend werden 50 ml einer wässrigen Formulierung der zu prüfenden Verbindung I (erhalten aus einem 25%igem Spritzpulver) jeweils in einer Konzentration von 400 ppm direkt auf die Erde aufgegossen.

Nach 24 Stunden werden auf die oberirdischen Pflanzenteile der behandelten Pflanzen Blattläuse der Spezies Myzus persicae gesetzt und die Pflanzen mit Plastikzylindern überdeckt, um die Blattläuse vor einer eventuellen Kontakt- oder Gaswirkung der Testsubstanz zu schützen.

Die Auswertung der erzielten %-Abtötung erfolgt 48 Stunden nach Versuchsbeginn. Pro Testsubstanz werden zwei Pflanzen, je eine in separaten Töpfen, verwendet. Der Versuch wird bei ca. 25° C und 60 % relativer Luftfeuchtigkeit durchgeführt.

Die Verbindungen Nr. 1.1, 3.1, 3.2, 3.3, 3.4, 3.5, 3.6, 3.7 und 3.8 zeigen gute Wirkung in obigem Test.

3.10 Insektizide Blattpenetrations-Wirkung: Aphis craccivora

In etwa 8 cm hohe Kunststoffbecher (Durchmesser etwa 6 cm) wird ein passender kleiner Zweig von Vicia faba gelegt, der mit Blattläusen der Spezies Aphis craccivora stark infiziert ist. Der Becher wird mit einem Kunststoffdeckel, der in der Mitte eine ausgestanzte Oeffnung von 2 cm Durchmesser aufweist, bedeckt. Auf die in dem Deckel befindliche Oeffnung wird ein Blatt einer Vicia faba-Pflanze gelegt, ohne dieses Blatt von der eingetopften Pflanze abzutrennen. Das Blatt wird dann mit einem zweiten Lochdeckel auf dem Becher über der Oeffnung des ersten Deckels fixiert. Von der Unterseite her, d.h. durch die Oeffnung des ersten Deckels hindurch, infizieren nun die in dem Becher befindlichen Blattläuse das obenliegende Blatt der Futterpflanze. Auf der Oberseite wird auf das Blatt eine wässrige Zubereitung des zu prüfenden Wirkstoffs in einer Konzentration von 400 ppm mittels eines Pinsels gleichmässig aufgetragen. Es wird geprüft, ob die einseitig auf die Oberseite des Blattes der Futterpflanze aufgetragene Testsubstanz in genügender Menge durch das Blatt hindurch auf dessen Unterseite diffundiert, um dort saugende Blattläuse abzutöten.

Der Versuch wird bei etwa 20°C und 60 % relativer Luftfeuchtigkeit durchgeführt. Die Auswertung auf %-Mortalität erfolgt 48 Stunden nach Wirkstoffapplikation.

Die Verbindungen Nr. 1.1, 3.1, 3.2, 3.3, 3.4, 3.5, 3.6, 3.7 und 3.8 zeigen gute Wirkung in obigem Test.

3.11 Insektizide Wirkung (systemisch-Wasser): Aphis craccivora

Erbsenkeimlinge, die 24 Stunden vor Beginn des Versuches mit den Blattläusen infestiert worden waren, werden in 20 ml einer wässrigen Brühe gestellt, die 400 ppm des zu prüfenden Wirkstoffes enthält. Die wässrige Brühe wird aus einem Emulsionskonzentrat oder einer benetzbaren Pulverzubereitung des betreffenden Wirkstoffes hergestellt und befindet sich in einem Gefäss, dass mit einem Löcher aufweisenden Plastikdeckel abgeschlossen ist. Die Wurzel der infestierten Erbsenpflanze wird jeweils durch ein Loch in dem Plastikdeckel in die Brühe geschoben. Das Loch wird dann mit Watte abgedichtet, um die Pflanze zu fixieren und einen eventuellen Einfluss der Gasphase aus der Brühe auszuschalten.

Der Versuch wird bei 20°C und 60°C relativer Luftfeuchtigkeit durchgeführt. Nach zwei Tagen wird auf die Anzahl der nicht mehr saugfähigen Testtiere im Vergleich zu unbehandelten Kontrollen bonitiert. Damit wird festgestellt, ob der über die Wurzel aufgenommene Wirkstoff, die Blattläuse an den oberen Pflanzenteilen abtötet.

Die Verbindungen Nr. 1.1, 3.1, 3.2, 3.3, 3.4, 3.5, 3.6, 3.7 und 3.8 zeigen im obigen Versuch eine gute systemische Wirkung gegen Insekten der Spezies Aphis craccivora.

3.12 Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5.5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Belichtungsperiode von 16 Stunden durchgeführt.

Die Verbindungen Nr. 1.1, 3.1, 3.2, 3.3, 3.4, 3.5, 3.6, 3.7 und 3.8 zeigen in diesem Test gute Wirkung.

## Patentansprüche

1. 1-Nitro-2,2-diaminoaethylenderivate der Formel I

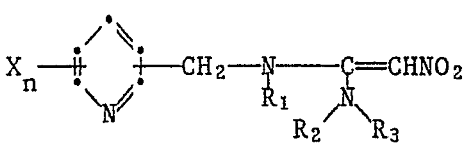

(I)

worin

X Chlor, Fluor, mit Halogen substituiertes oder unsubstituiertes $C_1$-$C_5$-Alkyl,
mit Halogen substituiertes oder unsubstituiertes $C_1$-$C_5$-Alkoxy,
mit Halogen substituiertes oder unsubstituiertes $C_1$-$C_5$-Alkylthio,
mit Halogen substituiertes oder unsubstituiertes Alkylsulphinyl,
mit Halogen substituiertes oder unsubstituiertes Alkylsulphonyl, ferner Nitro, Cyano, Thiocyanato, $C_3$-$C_5$-Haloalkenyl, $C_3$-$C_5$-Haloalkinyl, Hydroxy, $C_1$-$C_5$-Alkoxycarbonyl, Amino, Di-$C_1$-$C_4$-alkylamino, $C_1$-$C_5$-Alkylcarbonyl, $C_1$-$C_5$-Alkylcarbonylamino oder $C_1$-$C_5$-Alkylcarbonyloxy,
n die Zahlen 0-4,
$R_1$ Wasserstoff, $C_1$-$C_5$-Alkyl, $C_3$-$C_7$-Cycloalkyl,
$R_2$ Wasserstoff, $C_1$-$C_5$-Alkyl, $C_3$-$C_7$-Cycloalkyl,
$R_3$ Wasserstoff, $C_1$-$C_5$-Alkyl, $C_3$-$C_7$-Cycloalkyl, Benzyl oder Pyridinylmethyl, oder
$R_2$ und $R_3$ zusammen mit dem sie verbindenden Stickstoffatom der Pyrrolidinyl- oder den Piperazinylrest bedeuten, wobei nicht mehr als einer der Reste $R_1$, $R_2$ und $R_3$ für Wasserstoff steht, einschliesslich der cis- und trans-Isomere und einschliesslich der Salze der Verbindungen der Formel I.

2. Verbindungen gemäss Anspruch 1, worin X Chlor oder Fluor bedeutet.

3. Verbindungen gemäss Anspruch 2, worin
X Chlor,
n die Zahlen 0-2,
$R_1$ Wasserstoff, Methyl, Aethyl oder Cyclopropyl,
$R_2$ Wasserstoff oder Methyl,
$R_3$ Wasserstoff, $C_1$-$C_5$-Alkyl, Benzyl oder Pyridinylmethyl, oder
$R_2$ und $R_3$ zusammen mit dem sie verbindenden Stickstoffatom der Pyrrolidinyl- oder den N-Piperazinylrest, bedeuten.

4. Verbindungen gemäss Anspruch 3 der Formel Ia,

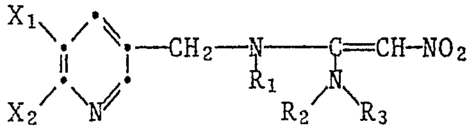

(Ia)

worin

$X_1$ und $X_2$ voneinander unabhängig Wasserstoff oder Chlor und $R_1$ Wasserstoff, Methyl oder Aethyl bedeuten.

5. Verbindungen gemäss Anspruch 4, worin n = 0 ist.

6. Verbindungen der Formel Ia gemäss Anspruch 4, worin $X_1$ und $X_2$ Chlor bedeuten.

7. Die Verbindung gemäss Anspruch 5 der Formel

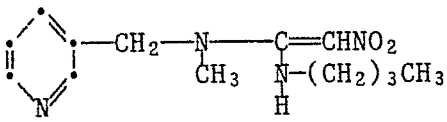

.

8. Die Verbindung gemäss Anspruch 5 der Formel

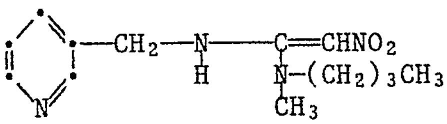

.

9. Die Verbindung gemäss Anspruch 5 der Formel

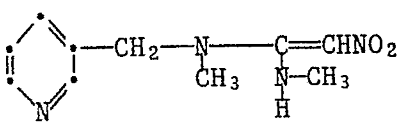

.

10. Die Verbindung gemäss Anspruch 5 der Formel

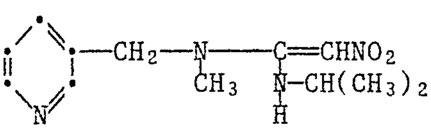

.

11. Die Verbindung gemäss Anspruch 5 der Formel

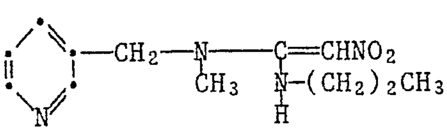

.

12. Die Verbindung gemäss Anspruch 5 der Formel

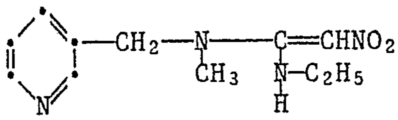 .

13. Die Verbindung gemäss Anspruch 5 der Formel

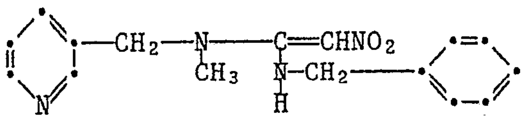 .

14. Die Verbindung gemäss Anspruch 5 der Formel

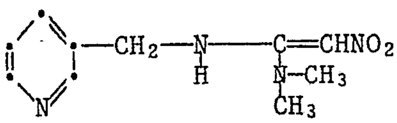 .

15. Die Verbindung gemäss Anspruch 5 der Formel

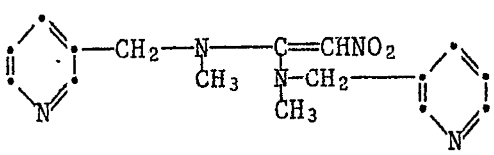 .

16. Die Verbindung gemäss Anspruch 5 der Formel

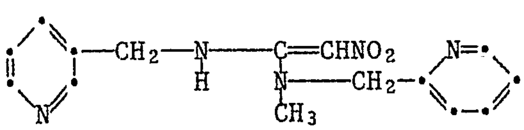 .

17. Die Verbindung gemäss Anspruch 5 der Formel

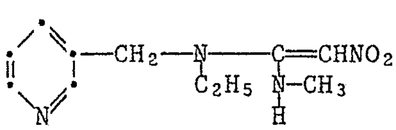 .

18. Die Verbindung gemäss Anspruch 6 der Formel

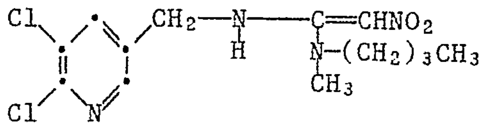

.

19. Die Verbindung gemäss Anspruch 3 der Formel Ic,

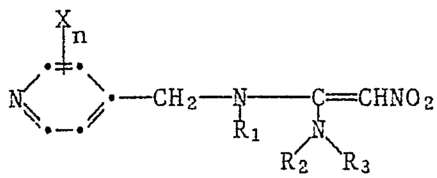

(Ic)

.

20. Verfahren zur Herstellung einer Verbindung der Formel I, Anspruch 1, dadurch gekennzeichnet, dass man ein Nitroäthylenderivat der Formel II

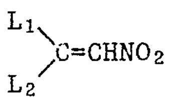

(II),

in welcher $L_1$ und $L_2$ voneinander unabhängig $C_1$-$C_4$-Alkylthio, $C_1$-$C_4$-Alkylsulfinyl oder Chlor bedeuten, stufenweise, entweder

a) zunächst mit der äquivalenten Menge von einem Pyridinylmethylamin der Formel III

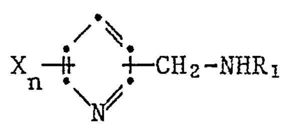

(III),

worin $R_1$ die unter Formel I angegebene Bedeutung hat, zum 1-Nitro-2-pyridinylmethylamino-äthylenderivat der Form IV

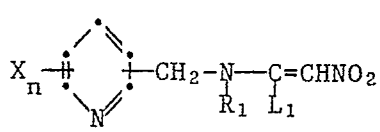

(IV),

worin $R_1$, X und n bzw. $L_1$ die unter den Formeln I bzw. II angegebene Bedeutung haben, umsetzt und anschliessend die Verbindung IV mit einem Amin der Formel V

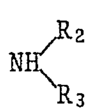

(V),

worin $R_2$ und $R_3$ die unter Formel I angegebene Bedeutung haben, zum 1-Nitro-2,2-diamino-äthylenderivat der Formel I umsetzt, oder

b) zunächst ein Nitroäthylenderivat der Formel II mit der äquivalenten Menge von einem Amin der

Formel V zum 1-Nitro-2-amino-äthylenderivat der Formel VI,

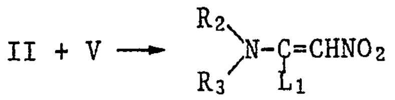

(VI),

worin $R_2$ und $R_3$, bzw. $L_1$ die unter den Formeln II bzw. V angegebene Bedeutung haben, umsetzt und anschliessend diese Verbindung mit einem Pyridinylmethylamin der Formel III zum 1-Nitro-2,2-diamino-äthylenderivat der Formel I umsetzt und dieses isoliert.

21. Verfahren gemäss Anspruch 20, dadurch gekennzeichnet, dass man die Zwischenprodukte der Formel IV oder der Formel IV, ohne Isolierung in die Verbindung der Formel I überführt.

22. Verfahren gemäss Anspruch 20, dadurch gekennzeichnet, dass man die Verbindung der Formel IV isoliert.

23. 1-Nitro-2-pyridinylmethylamino-äthylenderivate der Formel IV

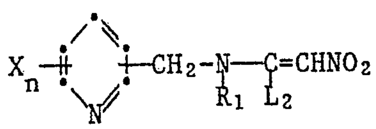

(IV),

worin $R_1$ die unter Formel I, Anspruch 1 und $L_2$ die unter Anspruch 20 angegebene Bedeutung haben.

24. Schädlingsbekämpfungsmittel, welches als aktive Komponente mindestens eine Verbindung der Formel I, gemäss Anspruch 1 oder mindestens eine Verbindung der Formel IV, gemäss Anspruch 20 enthalten, zusammen mit geeigneten Trägern und/oder Zuschlagstoffen.

25. Verwendung einer Verbindung der Formel I gemäss Anspruch 1 oder einer Verbindung der Formel IV, gemäss Anspruch 20 zur Bekämpfung von Schädlingen an Tieren und Pflanzen.

26. Verfahren zur Bekämpfung von Schädlingen an Tieren und Pflanzen, dadurch gekennzeichnet, dass man die Schädlinge in ihren verschiedenen Entwicklungstadien mit einer Verbindung der Formel I gemäss Anspruch 1 oder einer Verbindung der Formel IV, gemäss Anspruch 20 in Kontakt bringt.